# EUROPEAN PATENT APPLICATION

(11) **EP 3 431 088 A1**
(43) Date of publication of application: **23.01.2019**
(21) Application number: 17305970.0
(22) Date of filing: 20.07.2017
(51) Int. Cl.: A61K 31/555, A61K 45/06, A61K 9/00, A61P 3/10, A61P 31/18, A61P 37/04, A61P 37/06, A61P 43/00

(54) **MNTBAP FOR USE IN INDUCING A TRANSIENT IMMUNE TOLERANCE**

(71) Applicant: Paris Sciences et Lettres - Quartier Latin, 75006 Paris (FR); Ecole Pratique des Hautes Etudes, 75014 Paris (FR); INSERM (Institut National de la Santé et de la Recherche Médicale), 75654 Paris Cedex 13 (FR); GENETHON, 91000 Evry (FR)
(72) Inventor: NEILDEZ, Thi My Anh, 91230 Montgeron (FR)
(74) Representative: Icosa

(57) **Abstract**

The present invention relates to MnTBAP for use in inducing a transient immune tolerance to an antigen in a subject, wherein said transient immune tolerance is maintained for less than 2 months after the last administration of MnTBAP.

## Description

### FIELD OF INVENTION

The present invention relates to the induction of a transient immune tolerance to an antigen for suppressing, inhibiting or preventing unwanted immune responses, for example in autoimmune diseases, graft rejection, graft versus host disease (GVHD) or during gene therapy. Furthermore, this invention relates also to the prevention of human immunodeficiency virus (HIV) infection.

### BACKGROUND OF INVENTION

The CD4 glycoprotein is expressed on a particular subset of mature T cells. The CD4 glycoprotein is a coreceptor of the T cell receptor (TCR) and plays a key role in the recognition of antigens presented by the major histocompatibility complex (MHC) class II. Therefore, the expression of CD4 on T cells determines and optimizes the activation status of CD4⁺ T cells against a specific antigen in order to initiate and regulate cellular and humoral immune responses. Furthermore, the CD4 glycoprotein is a high affinity receptor for the viral glycoprotein gp120 of the HIV virus. Thus, CD4 expression on T cells facilitates the entry of the HIV virus into the cell.

Autoimmune diseases, graft rejection and graft versus host disease (GVHD) results from an excess of cellular and humoral immune responses against a specific antigen. Furthermore, during gene therapy, cellular and humoral immune responses against the transgene or against the delivery vector can occur and limit the chance of success of the therapy.

Therefore, an interesting therapeutical option in these diseases would be to limit T cell activation by a decrease of CD4 expression. Similarly, in HIV patients, a decrease of CD4 expression would prevent or limit the entry of the HIV virus into CD4⁺ T cells helping to prevent, limit or treat the infection.

Moreover, in order to limit the occurrence of side effects related to immunosuppression, said limitation of T cell activation should be only transient and no cell depletion should be induced.

The Inventors surprisingly showed that Mn (III) tetrakis (4-benzoic acid) porphyrin chloride (MnTBAP), a metalloporphyrin synthetic molecule, is able to induce a rapid and reversible internalization of the CD4 glycoprotein expressed on CD4⁺ T cells. In addition, the Inventors demonstrate that this novel effect of MnTBAP leads to the absence of IFNγ production, i.e. T cell response, by CD4⁺ and CD8⁺ T cells against a specific antigen during a transient period. Moreover, the Inventors demonstrate that MnTBAP is able to inhibit primary and memory humoral responses against a vector (in particular to decrease to production of specific IgG against the vector), thereby allowing an efficient readministration of the vector during gene therapy.

The present invention thus relates to MnTBAP as a new treatment for suppressing, inhibiting or preventing unwanted immune responses in autoimmune diseases, graft rejection, graft versus host disease (GVHD) or during gene therapy. In addition, the present invention also relates to MnTBAP as a new treatment for suppressing, inhibiting or preventing HIV infection.

### SUMMARY

The present invention relates to MnTBAP or analogs thereof for use in inducing a transient immune tolerance to an antigen in a subject, wherein said immune tolerance is maintained for less than 2 months after the last administration of MnTBAP.

In one embodiment, MnTBAP is administered during 1 to 5 days, preferably once a day during 3 or 4 days.

In one embodiment, a dose of MnTBAP ranging from about 1 to about 1000 mg/kg, preferably from about 50 to about 100 mg/kg, more preferably of about 80 mg/kg is to be administered to the subject.

In one embodiment, MnTBAP induces the reversible internalization of the CD4 molecules at the T cell membrane, thereby resulting in a transient immune tolerance.

In one embodiment, the subject is a human subject.

In one embodiment, said antigen is recognized by T cells and induces an unwanted immune response.

In one embodiment, said unwanted immune response is an autoimmune disease, graft rejection or graft versus host disease (GVHD).

In one embodiment, the subject is affected with an autoimmune disease, graft rejection or graft versus host disease (GVHD).

In one embodiment, said autoimmune disease is selected from the group comprising type 1 diabetes and autoimmune arthritis.

In one embodiment, the subject is an autoimmune disease patient exhibiting a flare up of said autoimmune disease.

In one embodiment, the subject is treated by gene therapy.

In one embodiment, the antigen is the vector or the transgene or the transgene product used in gene therapy.

Another object of the invention is MnTBAP or analogs thereof for use in preventing or treating HIV in a subject, wherein MnTBAP prevents contamination of non-infected CD4⁺ T cells by HIV particles.

In one embodiment, the subject is a HIV patient, and MnTBAP is administered in combination with a HIV treating agent.

In one embodiment, the subject is or will be in contact with the HIV virus, and MnTBAP administration prevents contamination of the subject by HIV virus.

### DEFINITIONS

In the present invention, the following terms have the following meanings:
- **"Treatment"** refers to both therapeutic treatment and prophylactic or preventative measures; wherein the object is to prevent or slow down (lessen) the targeted pathologic condition or disorder. Those in need of treatment include those already with the disorder as well as those prone to have the disorder or those in whom the disorder is to be prevented. A subject or mammal is successfully "treated" for the targeted pathologic condition or disorder if, after receiving a therapeutic amount of MnTBAP or of an analog thereof, the patient shows observable and/or measurable reduction in or absence of one or more of the following: reduction in the number of pathogenic cells; reduction in the percent of total cells that are pathogenic; relief to some extent of one or more of the symptoms associated with the targeted pathologic condition or disorder; reduced morbidity and mortality, and/or improvement in quality of life issues. The above parameters for assessing successful treatment and improvement in the disease are readily measurable by routine procedures familiar to a physician.
- **"Therapeutically effective amount"** refers to level or amount of agent that is aimed at, without causing significant negative or adverse side effects to the target, (1) delaying or preventing the onset of the targeted pathologic condition or disorder; (2) slowing down or stopping the progression, aggravation, or deterioration of one or more symptoms of the targeted pathologic condition or disorder; (3) bringing about ameliorations of the symptoms of the targeted pathologic condition or disorder; (4) reducing the severity or incidence of the targeted pathologic condition or disorder; (5) curing the targeted pathologic condition or disorder. An effective amount may be administered prior to the onset of the targeted pathologic condition or disorder, for a prophylactic or preventive action. Alternatively, or additionally, the effective amount may be administered after initiation of the targeted pathologic condition or disorder, for a therapeutic action.
- **"Pharmaceutically acceptable excipient"** refers to an excipient that does not produce an adverse, allergic or other untoward reaction when administered to an animal, preferably a human. It includes any and all dispersion media and solvents, coatings, isotonic and absorption delaying agents, additives, preservatives, stabilizers and the like. For human administration, preparations should meet sterility, pyrogenicity, general safety and purity standards as required by regulatory offices, such as, for example, FDA Office or EMA.

- **"Subject"** refers to a mammal, preferably a human. In one embodiment, a subject may be a "patient", i.e. a warm-blooded animal, more preferably a human, who/which is awaiting the receipt of, or is receiving medical care or was/is/will be the object of a medical procedure, or is monitored for the development of a disease. In one embodiment, the subject is an adult (for example a subject above the age of 18). In another embodiment, the subject is a child (for example a subject below the age of 18). In one embodiment, the subject is a male. In another embodiment, the subject is a female.
- **"About"** preceding a figure means plus or less 10% of the value of said figure.

### DETAILED DESCRIPTION

The present invention relates to MnTBAP or analogs thereof (in particular salts or solvates thereof) for use in inducing a transient immune tolerance to an antigen in a subject.

Indeed, the Applicant showed that the administration of MnTBAP results in the reversible internalization of CD4 on CD4⁺ T cells, thereby resulting in the induction of a transient immune tolerance to an antigen. As used herein, an "immune tolerance" refers to a state of unresponsiveness of the immune system to an antigen that has the capacity to elicit an immune response. One interesting advantage of MnTBAP is that the reversible CD4 internalization on CD4⁺ T cells does not induce any cell depletion.

In one embodiment, the administration of MnTBAP results in a reversible internalization of the CD4 molecules at the T cell membrane. As used herein, a "reversible internalization" is an internalization that can be returned to the prior condition, i.e. the prior CD4 expression on CD4⁺ T cells.

In one embodiment, the said reversible internalization leads to a transient immune tolerance. In one embodiment, said immune tolerance is maintained for less than 1 year, preferably less than 6 months, more preferably less than 4 months and even more preferably less than 2 months after the last administration of MnTBAP.

MnTBAP is defined as manganese [III] tetrakis (4-benzoic acid) porphyrin. Another commonly used name for MnTBAP is manganese [III] tetrakis (5, 10, 15, 20 benzoic acid) porphyrin. In its solid state, MnTBAP is a chloride salt, but in aqueous and biological conditions, MnTBAP is a positively charged compound. MnTBAP, which does not occur naturally in mammalian cells, is a small synthetic molecule that does not cross the blood-brain barrier.

In one embodiment, the analog of MnTBAP is a salt of MnTBAP, preferably a pharmaceutically acceptable salt of MnTBAP. Examples of pharmaceutically acceptable salts include, but are not limited to, salts with inorganic bases, salts with organic bases, salts with inorganic acids, salts with organic acids, salts with basic or acidic amino acids and the like. Examples of salts with an inorganic base include, but are not limited to, alkali metal salts, such as a sodium salt and a potassium salt; an alkaline earth metal salt such as a calcium salt and a magnesium salt; an aluminum salt; and an ammonium salt. Examples of salts with an organic base include, but are not limited to, salts with trimethylamine, triethylamine, pyridine, picoline, 2,6-lutidine, ethanolamine, diethanolamine, triethanolamine, cyclohexylamine, dicyclohexylamine and N,N'-dibenzylethylenediamine. Examples of salts with an inorganic acid include, but are not limited to, salts with hydrochloric acid, boric acid, nitric acid, sulfuric acid and phosphoric acid. Examples of salts with an organic acid include, but are not limited to, salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid and p-toluenesulfonic acid. Examples of salts with a basic amino acid include, but are not limited to, salts with arginine, lysine and ornithine. Examples of salts with an acidic amino acid include, but are not limited to, salts with aspartic acid and glutamic acid. A non-exhaustive list of suitable salts is disclosed in Remington's Pharmaceutical Sciences, 17th ed., Mack Publishing Company, Easton, Pa., p 1418, 1985, the entire disclosure of which is incorporated herein by reference.

In another embodiment, the analog of MnTBAP is a solvate of MnTBAP. As referred herein, "solvate" describes a molecular complex comprising the compound MnTBAP and one or more pharmaceutically acceptable solvent molecules, for example, ethanol. The term "hydrate" is employed when said solvent is water.

In another embodiment, analogs may include a manganese porphyrin related to MnTBAP, for example, Mn [III] tetra(4-pyridyl) porphyrin (MnTyP) and Mn [III] Meso-Tetrakis-(N-Methylpyridinium-2-yl) porphyrin. Analogs may further include porphyrin molecules containing other metal cations such as Iron(III) Tetrakis (4 Nmethylpyridyl) porphyrin or similar molecules without a metal cation.

As used herein, the term "antigen" refers to a molecule that provokes an immune response. This immune response may involve either antibody production (i.e. the immune response is a humoral immune response), or the activation of specific immunologically-competent cells (i.e. the immune response is a cellular immune response), or both. The skilled artisan will understand that any macromolecule, including virtually all proteins or peptides, can serve as an antigen. Furthermore, antigens can be derived from recombinant or genomic DNA. A skilled artisan will understand that any DNA, which comprises a nucleotide sequence or a partial nucleotide sequence encoding a protein that elicits an immune response therefore encodes an "antigen" as that term is used herein. It is readily apparent that an antigen can be synthesized or can be derived from a biological sample, or might be macromolecule besides a polypeptide. Such a biological sample can include, but is not limited to a tissue sample, a cell or a fluid with other biological components.

In particular embodiments, the antigen induces an unwanted immune response. In one embodiment, the unwanted immune response results from an autoimmune disease, graft rejection, graft versus host disease (GVHD) and/or a gene transfer.

In one embodiment, the antigen is an autoantigen.

Examples of autoantigens include, but are not limited to, non-specific islet cell autoantigens (ICA), insulin, glutamic acid decarboxylase 65 (GAD65), insulinoma antigen-2 (IA-2), heat shock protein (HSP), islet-specific glucose-6-phosphatase catalytic subunit related protein (IGRP), imogen-38, zinc transporter-8 (ZnT8), pancreatic duodenal homeobox factor 1 (PDX1), chromogranin A (CHGA), islet amyloid polypeptide (IAPP), proteolipid protein, 2',3'-Cyclic-nucleotide 3'-phosphodiesterase (CNPase), S100β protein, transaldolase H, immunoglobulin G, Fc-part of immunoglobulins, carbamylated antigens, cartilage glycoprotein-39, type II collagen, human chondrocyte glycoprotein 39, proteoglycan, Epstein-Barr virus trans-acting factor, *Escherichia coli* dnaJ, citrullinated proteins such as for example citrullinated filaggrin, aquaporin-4 water channel (AQP4), Hu, Ma2, collapsin response-mediator protein 5 (CRMP5), and amphiphysin, voltage-gated potassium channel (VGKC), N-methyl-d-aspartate receptor (NMDAR), α-amino-3-hydroxy-5-methyl-4-isoxazoleproprionic acid (AMPAR), thyroid peroxidase, thyroglobulin, Anti-N-methyl-D-aspartate receptor (NR1 subunit), Rh blood group antigens, I antigen, Dsg1/3, BP180, BP230, Acetylcholine nicotinic postsynaptic receptors, thyrotropin receptors, platelet integrin, GpIIb:IIIa, Collagen alpha-3(IV) chain, rheumatoid factor, calpastatin, Myelin basic protein (MBP), Myelin oligodendrocyte glycoprotein (MOG) peptides, alpha-beta-crystallin, DNA, histone, ribosomes, RNP, tissue transglutaminase (TG2), intrinsic factor, 65-kDa antigen, phosphatidylserine, ribosomal phosphoproteins, anti-neutrophil cytoplasmic antibody, Scl-70, U1-RNP, ANA, SSA, anti-SSB, antinuclear antibodies (ANA), antineutrophil cytoplasm antibodies (ANCA), Jo-1, antimitochondrial antibodies, gp210, p62, sp100, antiphospholipid antibodies, U1-70 kd snRNP, GQlb ganglioside, GM1, asialo GM1, GD1b, anti-smooth muscle antibodies (ASMA), anti-liver-kidney microsome-I antibodies (ALKM-1), anti-liver cytosol antibody-1 (ALC-1), IgA antiendomysial antibodies, neutrophil granule proteins, streptococcal cell wall antigen, intrinsic factor of gastric parietal cells, PLA2R1 and THSD7A1.

In another embodiment, the antigen is the vector or the transgene or the transgene product (i.e. the product encoded by the transgene) used in gene therapy.

The present invention relates to a composition for use in inducing a transient immune tolerance to an antigen in a subject comprising, consisting or consisting essentially of MnTBAP or analogs thereof.

As used herein, "consisting essentially of", with reference to a composition, means that MnTBAP or analogs thereof is the only one therapeutic agent or agent with a biologic activity within said composition.

In one embodiment, the composition for use in the present invention does not include a selenium compound. The term "selenium compound" encompasses the element selenium and sources of the element (e.g., dietary sources such as cereals, grains and vegetables) as well as compounds that contain selenium. Examples of selenium compounds include, but are not limited to, sodium selenite, selenomethionine, methylselenocysteine, 2-methyl-selenoazolidine, selenobetaine methyl ester, selenocysteine, selenobetaine, and selenoazolidine.

Another object of the invention is a pharmaceutical composition for use in inducing a transient immune tolerance to an antigen in a subject comprising, consisting or consisting essentially of MnTBAP or analogs thereof and at least one pharmaceutically acceptable excipient. In one embodiment, the pharmaceutical composition for use in the present invention does not include a selenium compound as defined herein above.

Suitable excipients include water, saline, Ringer's solution, dextrose solution, and solutions of ethanol, glucose, sucrose, dextran, mannose, mannitol, sorbitol, polyethylene glycol (PEG), phosphate, acetate, gelatin, collagen, Carbopol®, vegetable oils, and the like. One may additionally include suitable preservatives, stabilizers, antioxidants, antimicrobials, and buffering agents, such as, for example, BHA, BHT, citric acid, ascorbic acid, tetracycline, and the like.

Other examples of pharmaceutically acceptable excipients that may be used in the composition of the invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene- polyoxypropylene- block polymers, polyethylene glycol and wool fat.

In one embodiment, the composition or the pharmaceutical composition of the invention may comprise some excipients, such as, for example, surfactants (e.g. hydroxypropylcellulose); suitable carriers, such as, for example, solvents and dispersion media containing, for example, water, ethanol, polyol (e.g. glycerol, propylene glycol, and liquid polyethylene glycol, and the like), suitable mixtures thereof, and vegetable oils, such as, for example, peanut oil and sesame oil; isotonic agents, such as, for example, sugars or sodium chloride; coating agents, such as, for example, lecithin; agents delaying absorption, such as, for example, aluminum monostearate and gelatin; preservatives, such as, for example, benzalkonium chloride, benzethonium chloride, chlorobutanol, thimerosal and the like; buffers, such as, for example, boric acid, sodium and potassium bicarbonate, sodium and potassium borates, sodium and potassium carbonate, sodium acetate, sodium biphosphate and the like; tonicity agents, such as, for example, dextran 40, dextran 70, dextrose, glycerin, potassium chloride, propylene glycol, sodium chloride; antioxidants and stabilizers, such as, for example, sodium bisulfite, sodium metabisulfite, sodium thiosulfite, thiourea and the like; nonionic wetting or clarifying agents, such as, for example, polysorbate 80, polysorbate 20, poloxamer 282 and tyloxapol; viscosity modifying agents, such as, for example dextran 40, dextran 70, gelatin, glycerin, hydroxyethylcellulose, ydroxmethylpropylcellulose, lanolin, methylcellulose, petrolatum, polyethylene glycol, polyvinyl alcohol, polyvinylpyrrolidone, carboxymethylcellulose; and the like.

Another object of the invention is a medicament for use in inducing a transient immune tolerance to an antigen in a subject comprising, consisting or consisting essentially of MnTBAP or analogs thereof.

In one embodiment, the medicament for use in the present invention does not include a selenium compound as defined herein above.

The present invention further relates to MnTBAP or analogs thereof for use in treating autoimmune diseases, graft rejection, graft versus host disease (GVHD) or HIV infection.

In one embodiment, MnTBAP or analogs thereof, or the composition, pharmaceutical composition or medicament of the invention is for treating graft rejection or GVHD.

In one embodiment, the subject is affected with graft rejection.

In one embodiment, the subject is affected with graft versus host disease (GVHD).

In one embodiment, MnTBAP or analogs thereof, or the composition, pharmaceutical composition or medicament of the invention is for treating an autoimmune disease in a subject in need thereof. According to this embodiment, immune tolerance is induced to the autoantigen involved in the autoimmune disease to be treated.

Examples of autoimmune diseases include, but are not limited to, type 1 diabetes, autoimmune arthritis, rheumatoid arthritis, lupus erythematosus, disseminated lupus erythematosus, systemic scleroderma, multiple sclerosis, autoimmune haemolytic anaemias, autoimmune thrombopenia, polymyositides, dermatomyositides, vesicular pemphigus or pemphigus vulgaris, psoriasis, Berger disease, Basedow disease, Hashimoto thyroiditis, primary myxoedema, coeliac disease, Crohn's disease, herpetiform dermatitis, myasthenia, haemorrhagic rectocolitis, primary biliary cirrhosis, primary sclerosing cholangitis, Biermer anaemia, CREST syndrome, acquired epidermolysis bullosa, Lambert-Eaton myasthenic syndrome, polymyositis, Goujerot-Sjogren syndrome, Guillain-Barre syndrome, optical neuritis, psoriasis, bone-marrow aplasia, Reiter syndrome, primary biliary cirrhosis, antiphospholipid antibody syndrome, opsoclonus-myoclonus syndrome, temporal arteritis, acute disseminated encephalomyelitis, Goodpasture syndrome, Wegener granulomatosis, Churg-Strauss syndrome, sarcoidosis, nephrotic syndrome and La Peyronie disease.

In one embodiment, the autoimmune disease is type 1 diabetes. Examples of autoantigens involved in type 1 diabetes include, but are not limited to, non-specific islet cell autoantigens (ICA), insulin, glutamic acid decarboxylase 65 (GAD65), insulinoma antigen-2 (IA-2), heat shock protein (HSP), islet-specific glucose-6-phosphatase catalytic subunit related protein (IGRP), imogen-38, zinc transporter-8 (ZnT8), pancreatic duodenal homeobox factor 1 (PDX1), chromogranin A (CHGA), islet amyloid polypeptide (IAPP), proteolipid protein, 2',3'-Cyclic-nucleotide 3'-phosphodiesterase (CNPase), S100β protein, transaldolase H, Fc-part of immunoglobulins, carbamylated antigens, and cartilage glycoprotein-39.

In one embodiment, the autoimmune disease is autoimmune arthritis. Examples of autoantigens involved in autoimmune arthritis include, but are not limited to, type II collagen, human chondrocyte glycoprotein 39, proteoglycan, heat-shock proteins, Epstein-Barr virus trans-acting factor, *Escherichia coli* dnaJ, citrullinated filaggrin and immunoglobulin G.

In one embodiment, the subject is an autoimmune disease patient. In one embodiment, the subject is an autoimmune disease patient exhibiting a flare up of said autoimmune disease.

In one embodiment, MnTBAP or an analog thereof is administered during a flare-up of the autoimmune disease.

In one embodiment, MnTBAP or an analog thereof is administered during a remission stage (i.e., between flare-up stages of the autoimmune disease), wherein little or no symptoms are experienced by the patient. In one embodiment, MnTBAP or an analog thereof is administered to the subject just before a flare-up of the autoimmune disease is expected, such as, for example, 3 months, 1 month, 2 weeks, 1 week, 3 days, 2 days or 1 day before a flare-up of the autoimmune disease is expected.

In one embodiment, the administration of MnTBAP or of an analog thereof during a remission stage allows preventing a flare-up of the autoimmune disease.

The present invention further relates to MnTBAP or analogs thereof for use in preventing an unwanted immune response against the transgene or against the delivery vector during gene therapy in a subject in need thereof.

As used herein "gene therapy" or "gene transfer" are methods or systems for reliably inserting foreign DNA or the transgene into host cells. Such methods can result in transient expression of non-integrated transferred DNA, extrachromosomal replication and expression of transferred replicons (e.g. episomes), or integration of transferred genetic material into the genomic DNA of host cells.

Typically, viral vectors carry the transgene. Examples of viral vector include, but are not limited to, adenoviral, retroviral, lentiviral, herpesvirus and adeno-associated virus (AAV) vectors.

By an "AAV vector" is meant a vector derived from an adeno-associated virus serotype, including without limitation, AAV-1, AAV-2, AAV-3, AAV-4, AAV-5, AAV6, etc.

In one embodiment, the subject is treated by gene therapy.

In one embodiment, MnTBAP or analogs thereof, or the composition, pharmaceutical composition or medicament of the invention is for preventing HIV infection in a subject in need thereof, or for preventing infection of non-infected lymphocytes in a HIV patient.

In another embodiment, the subject is a HIV patient.

In one embodiment, the subject is or will be in contact with the HIV virus. In one embodiment, MnTBAP or analogs thereof administration prevents contamination of the said subject in contact with the HIV virus.

In one embodiment, the administration of MnTBAP or of an analog thereof results in a reversible internalization of the CD4 molecules at the T cell membrane, thereby preventing contamination of non-infected CD4⁺ T cells by HIV particles.

MnTBAP or analogs thereof, the composition, the pharmaceutical composition or the medicament may be administered via an oral, intravenous, rectal, vaginal, topical, transmucosal, intestinal, parenteral, intrathecal, intraventricular, intraperitoneal, transdermal, subcutaneous capsular dispersion (implant), intranasal, inhalation spray or intraocular administration route. In one embodiment, MnTBAP or analogs thereof, the composition, the pharmaceutical composition or the medicament of the present invention is administered intraperitoneally.

In one embodiment, MnTBAP or analogs thereof, the composition, the pharmaceutical composition or the medicament of this invention is in a form suitable for parenteral administration. Forms suitable for parenteral administrations include, but are not limited to, sterile isotonic aqueous or non-aqueous solutions, dispersions, suspensions or emulsions, or sterile powders which may be reconstituted into sterile injectable solutions or dispersions just prior to use.

In another embodiment, MnTBAP or analogs thereof, the composition, the pharmaceutical composition or the medicament of the present invention is in a form adapted to oral administration. According to a first embodiment, the form adapted to oral administration is a solid form selected from the group comprising tablets, pills, capsules, soft gelatin capsules, sugarcoated pills, orodispersing tablets, effervescent tablets or other solids. According to a second embodiment, the form adapted to oral administration is a liquid form, such as, for example, a drinkable solution, a buccal spray, liposomal forms and the like.

In another embodiment, MnTBAP or analogs thereof, the composition, the pharmaceutical composition or the medicament of the present invention is formulated for rectal or vaginal administration and may be presented as suppositories, pessaries, tampons, creams, gels, pastes, foams or sprays.

In another embodiment, MnTBAP or analogs thereof, the composition, the pharmaceutical composition or the medicament of the invention is in a form adapted for local delivery via the nasal and respiratory routes. Examples of formulations suitable for nasal or respiratory administration include, but are not limited to, nasal solutions, sprays, aerosols and inhalants.

In another embodiment, MnTBAP or analogs thereof, the composition, the pharmaceutical composition or the medicament of the invention is in a form adapted to a topical administration. Examples of formulations adapted to a topical administration include, but are not limited to, ointment, paste, eye drops, cream, patch, such as, for example, transdermal patch, gel, liposomal forms and the like.

In one embodiment, MnTBAP or analogs thereof is formulated at physiological pH, such as, for example, pH7, in a saline solution, such as for example PBS, or any other similarly suitable fluid at a concentration ranging from about 1 to about 50 mg/mL, preferably from about 5 to about 10 mg/mL, more preferably of about 8 mg/mL.

In one embodiment of the invention, MnTBAP or analogs thereof, the composition, the pharmaceutical composition or the medicament is administered at least once a month, preferably at least twice a month, more preferably at least once a week, and even more preferably at least once a day.

In one embodiment of the invention, MnTBAP, the composition, the pharmaceutical composition or the medicament is administered during 1 to 10 days, preferably during 1 to 5 days, more preferably during 2 to 4 days, even more preferably during 3 or 4 days.

In one embodiment of the invention, MnTBAP or analogs thereof, the composition, the pharmaceutical composition or the medicament is administered at least once a month, preferably at least twice a month, more preferably at least once a week, and even more preferably at least once a day during 1 to 10 days, preferably during 1 to 5 days, more preferably during 2 to 4 days, even more preferably during 3 or 4 days.

In one embodiment, a therapeutically effective amount of MnTBAP or analogs thereof, the composition, the pharmaceutical composition or the medicament of the present invention is administered to the subject.

In one embodiment of the invention, the dose of MnTBAP or analogs thereof administered to the subject ranges from about 1 to about 1000 mg/kg, preferably from about 50 to about 100 mg/kg, more preferably of about 80 mg/kg.

In one embodiment of the invention, the dose of MnTBAP or analogs thereof administered to the subject ranges from about 1 to about 100 mg/kg, preferably from about 2.5 to about 85 mg/kg, more preferably from about 10 to about 50 mg/kg.

It will be understood that the total daily usage of MnTBAP or analog thereof, composition, pharmaceutical composition or medicament of the present invention will be decided by the attending physician within the scope of sound medical judgment. The specific therapeutically effective dose level for any particular patient will depend upon a variety of factors including the disease being treated and the severity of the disease; activity of the specific compound or composition employed, the age, body weight, general health, sex and diet of the subject; the time of administration, route of administration, and rate of excretion of the specific compound employed; the duration of the treatment; drugs used in combination or coincidental with the specific compound employed; and like factors well known in the medical arts.

In one embodiment of the invention, MnTBAP or an analog thereof, the composition, the pharmaceutical composition or the medicament of the present invention is administered in combination with other treatments. Examples of other treatments include, but are not limited to, autoimmune disease treating agents, graft rejection treating agents, graft versus host disease (GVHD) treating agents or HIV treating agents.

Examples of autoimmune disease treating agents, graft rejection treating agents or graft versus host disease (GVHD) treating agents include, but are not limited to, immunosuppressant treatments, such as ciclosporin A, tacrolimus, sirolimus, inhibitor of TOR protein kinase, mycophenolic acid, a glucocorticoid, methotrexate, cyclophosphamide, azathioprine, sulphasalazine, an anti-TNF antibody, an anti-CD20 antibody, and anthymocyte globulin.

Examples of "HIV treating agents" include, but are not limited to, antiretroviral agents, such as, for example, Combivir, Kaletra, Trizivir, Kivexa, Truvada, Atripla, Eviplera Stribild, Triumeq, Evotaz Dutrebis, Genvoya, and Descovy.

The present invention further relates to a method for inducing a transient immune tolerance to an antigen in a subject in need thereof, wherein said method comprises administering MnTBAP or an analog thereof to the subject.

In one embodiment, the method of the invention is for treating autoimmune diseases, graft rejection, graft versus host disease (GVHD) or HIV infection.

In one embodiment, the method of the invention is for inducing the reversible internalization of the CD4 molecules at the T cell membrane, preferably through a clathrin mediated endocytosis mechanism. Indeed, the Inventors show herein that the administration of MnTBAP leads to the reversible internalization of the CD4 molecules at the T cell membrane, and that this internalization is dependent on a clathrin mediated-endocytosis mechanism.

In one embodiment, the method of the invention is for inducing the temporarily *in vivo* inhibition of specific CD4⁺ and/or CD8⁺ T-cell immune responses.

In one embodiment, the subject is treated by gene therapy (such as, for example, by intravenous or intramuscular rAAV gene transfer), and the method of the invention is for preventing CD8⁺ T cell infiltrate resulting from gene transfer.

In one embodiment, the subject is treated by gene therapy comprising intramuscular rAAV gene transfer, and the method of the invention is for preventing muscle destruction, resulting from gene transfer.

In another embodiment, the subject is treated by gene therapy (such as, for example, by intravenous or intramuscular rAAV gene transfer), and the method of the invention is for decreasing transgene-specific CD8⁺ and/or CD4⁺ T-cell responses induced by rAAV gene transfer, thereby resulting in a prolonged transgene expression in the subject.

In another embodiment, the subject is treated by gene therapy (such as, for example, by intravenous or intramuscular rAAV gene transfer), and the method of the invention is for inhibiting primary and memory humoral responses against the vector capsid or against the transgene or against the product encoded by the transgene, thereby allowing an efficient readministration of rAAV vector in the muscle. Indeed, the Applicant showed that the administration of MnTBAP results in a decrease in the production of specific anti-rAAV IgG post vector injection and post vector readministration. The method according to the present invention thus allows, in one embodiment, increasing significantly the number of copies of the vector (e.g. rAAVl) that may be readministered to the subject.

In another embodiment, the subject is treated by gene therapy (such as, for example, by intravenous or intramuscular rAAV gene transfer), and the method of the invention is for reducing inflammation induced by AAV-mediated gene transfer and/or for reducing the absolute number of splenic T-lymphocyte subsets, i.e. CD4 and/or CD8 T cell subsets.

In one embodiment, the method according to the present invention is for inducing a rapid disappearance of CD4 at the cell surface of CD4 expressing cells.

In one embodiment, the method of the present invention is for decreasing the infection efficiency of human T cells by the HIV virus. Indeed, the Applicant showed that the administration of MnTBAP induced a significant decrease of the transduction efficiency of human T cells by lentiviral vectors pseudotyped with HIV-1 envelope protein.

The present invention further relates to a method to blindfold temporarily a normally reactive immune system (including humoral and cellular immune responses) comprising administering MnTBAP to the subject.

The present invention further relates to a method to establish short-term blockade of immune responses to an antigen comprising administering MnTBAP to the subject.

The present invention further relates to a method to prevent remembrance by reducing the formation of immunological memory (e.g. the induction of memory cells) after initial antigen exposure comprising administering MnTBAP to the subject.

### BRIEF DESCRIPTION OF THE DRAWINGS

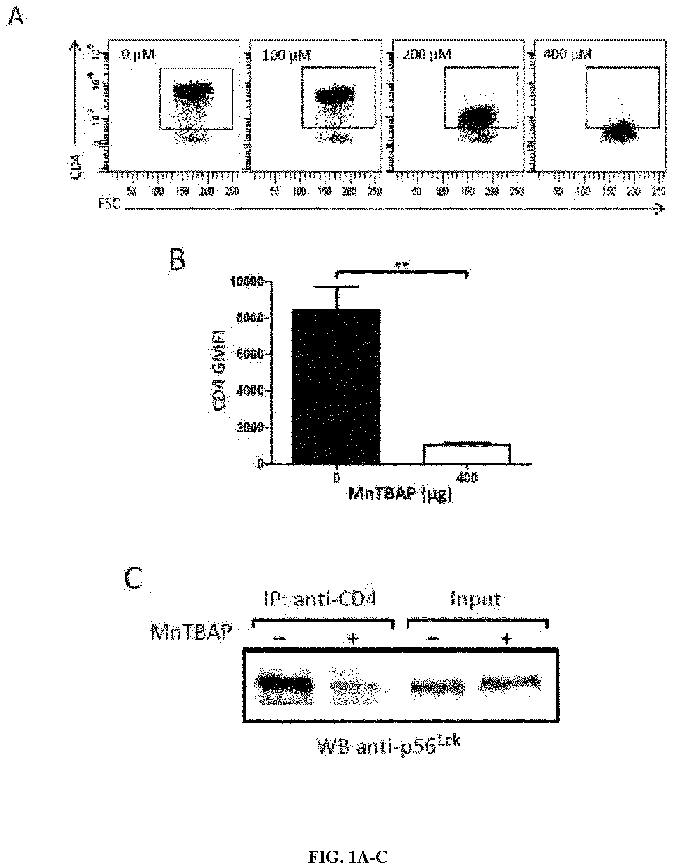

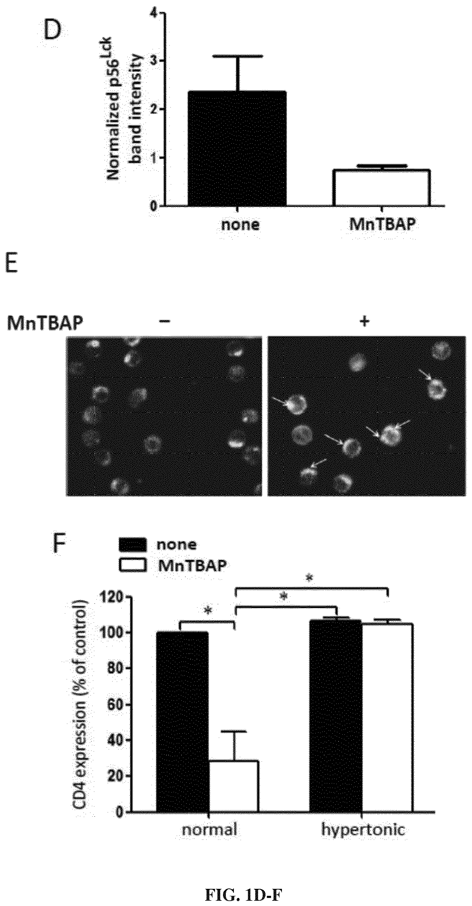

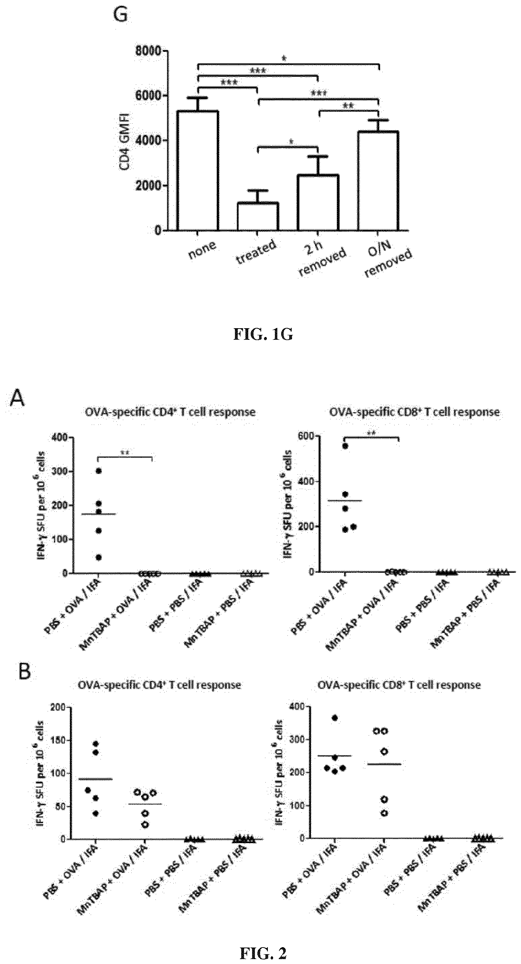

**Figure 1****:** MnTBAP-induced reversible CD4 internalization mechanism. **(A)** Representative flow cytometry dot plots showing progressive decrease of CD4 expression on viable murine splenic enriched CD4⁺ T cells treated in complete medium for 2 hours with increasing doses of MnTBAP. **(B)** Graph indicating CD4 GMFI (geometric mean fluorescence intensity) on enriched CD4⁺ T cells treated for 2 hours with or without MnTBAP at 400 µM. **(C)** Immunoprecipitation showing link disruption between CD4 and p56. **(D)** Graph of p56^{Lck} band detection normalized with the corresponding band in the input. **(E)** CD4 internalization by a clathrin mediated-endocytosis. Murine splenic enriched CD4⁺ T cells are cultured in absence or presence of 400 µM MnTBAP for 2 hours, and then cells are collected and subjected to immunostaining with anti-CD4 and anti-clathrin antibodies. Images are acquired with confocal-laser-scanning microscope. Arrows indicate colocalization of CD4 and clathrin. **(F)** Proof of clathrin mediated-endocytosis. Murine splenic enriched CD4⁺ T cells are cultured in absence or presence of 400 µM MnTBAP for 2 hours in either complete medium (RPMI) or hypertonic sucrose medium (RPMI + 0.45 M sucrose) known to inhibit specifically clathrin-dependent endocytosis. Cells are then collected and subjected to CD4 cell surface expression analysis by flow cytometry. Results are expressed as CD4 expression relative to control (RPMI without MnTBAP, "normal"), defined as 100%. **(G)** MnTBAP-induced CD4 internalization is reversible. Murine splenic enriched CD4⁺ T cells are cultured in absence or presence of 400 µM MnTBAP for 2 hours. Cells are then fixed (conditions "none" or "treated"), or either washed to remove MnTBAP and put back in culture for additional 2 hours (condition "2 h removed") or overnight (condition "O/N removed") and finally fixed. Fixed cells in all conditions are analyzed by flow cytometry for their CD4 expression, reported on the graph as CD4 GMFI.
**Figure 2****:** MnTBAP inhibits *in vivo* temporarily specific T-cell immune responses against OVA peptides. Female C57BL/6 mice are IP multi-injected with MnTBAP (80 mg/kg daily during 5 days) or with an equivalent volume of PBS. Two hours **(A)** or 21 days **(B)** post first IP injection of MnTBAP (open symbols) or PBS (closed symbols), mice are SC immunized with 100 µg of OVA peptides (circle symbols) or PBS (triangle symbols) complexed with IFA. Spleen cells are harvested 8 days after immunization to measure CD4⁺ and CD8⁺ T-cell responses by IFN-γ ELISPOT, following *in vitro* stimulation with OVA₃₂₃₋₃₃₉ and OVA₂₅₇₋₂₆₄ peptides, respectively. In each figure, left and right panels represent OVA-specific CD4⁺ and CD8⁺ T-cell responses, respectively. Each symbol represents IFN-γ spot-forming unit (duplicate measures) of each mouse. Horizontal bars indicate the average value. Statistical analyses are performed using Mann-Whitney test. **P < 0.005. OVA, chicken egg albumin; IP, intraperitoneally; PBS, phosphate-buffered saline; IFA, Incomplete Freund's Adjuvant; SC, subcutaneously; ELISPOT, enzyme-linked immunospot assay.

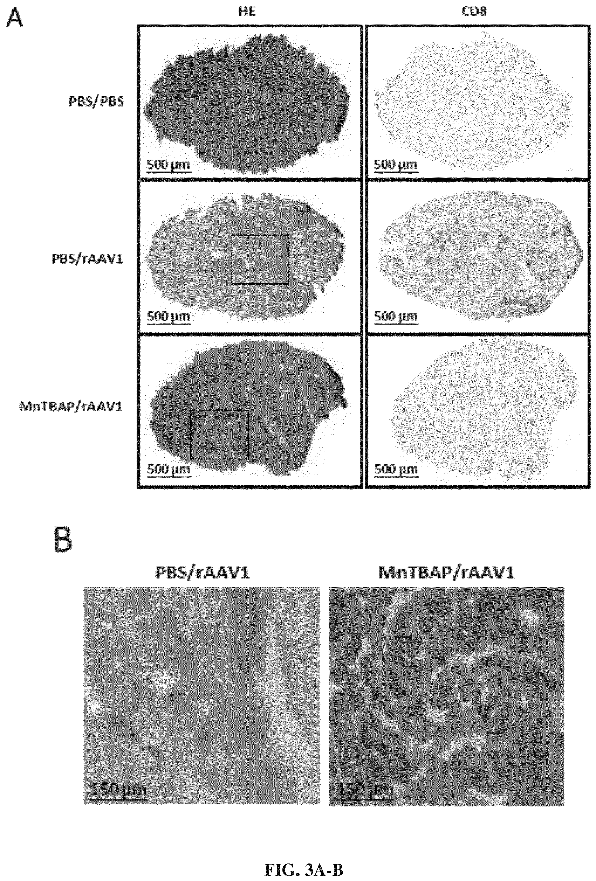

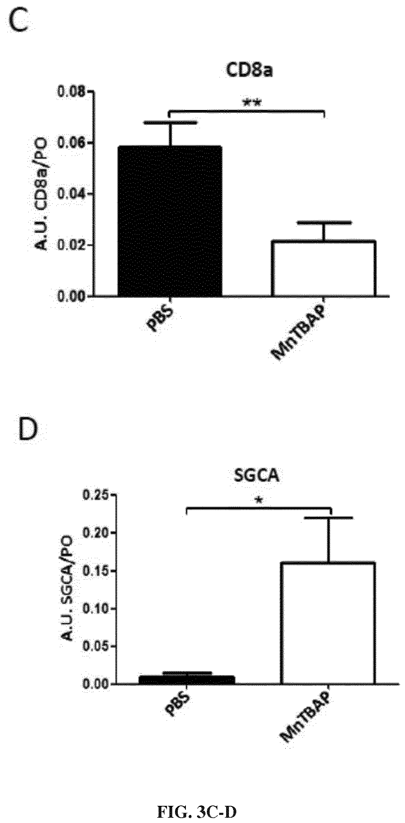

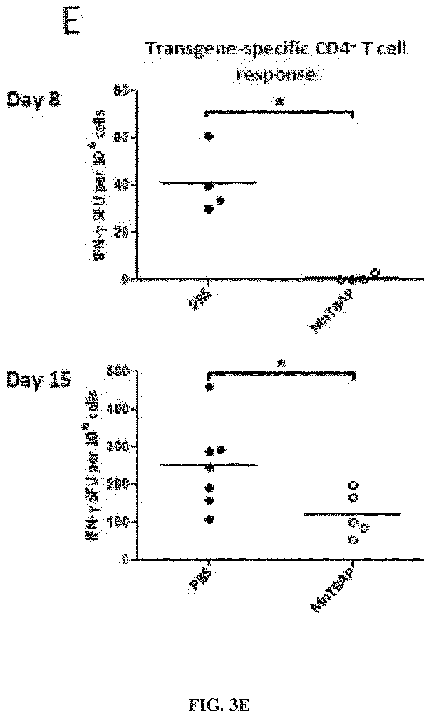

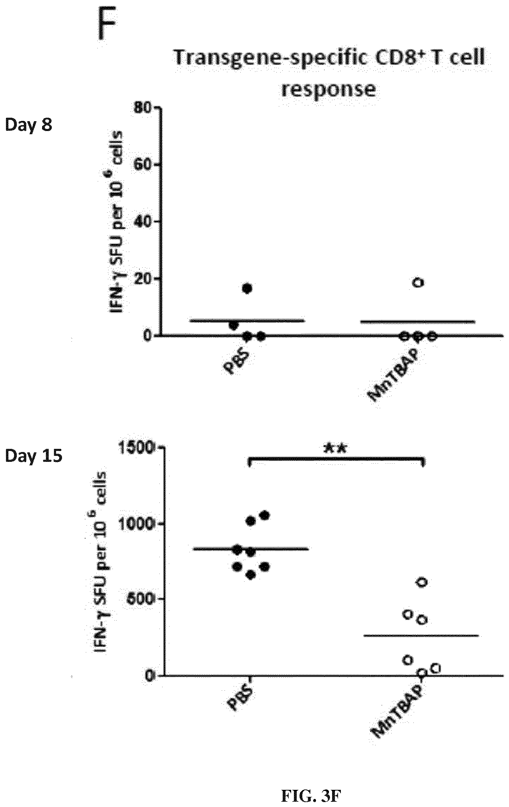

**Figure 3****:** MnTBAP prevents muscle destruction, CD8⁺ T cell infiltrate and prolongs transgene expression by decreasing transgene-specific CD8⁺ and CD4⁺ T-cell responses induced by IM rAAV gene transfer. Female C57BL/6 mice are IP multi-injected with MnTBAP (80 mg/kg daily during five days) or with an equivalent volume of PBS. Two hours post first injection of MnTBAP, mice are administrated with PBS or rAAV1 CMV SGCAHY vector (2.5.10⁹ vg per mouse) in the left TA. Fifteen days post rAAV vector injection, muscle integrity, transgene and CD8a mRNA expression are analyzed. **(A)** Histological analysis of injected TA muscle from PBS or MnTBAP treated mice, 15 days following injection of rAAV1 vector or control PBS. Microscopy analysis of cryosections of rAAV injected muscles after HE staining (left panels) and anti-CD8 staining followed by enzymatic detection (right panels) was performed. Images are representative of one experiment out of 2 with 3-4 mice per group. Scale bar = 500 µm. **(B)** High magnifications of selected areas shown in **(A).** Scale bar = 150 µm. **(C** and **D)** Analysis of CD8a and SGCA transgene expression by RT-PCR in injected TA muscle. Levels of CD8a (C) and SGCA **(D)** mRNA are expressed in arbitrary units (AU) relative to the endogenous murine acidic ribosomal phosphoprotein (PO) mRNA (n=2, 3-4 mice per group). **(E** and **F)** Analysis of transgene-specific T-cell responses. Eight (upper panels) and 15 days (lower panels) post injection of vector, spleen cells are harvested to measure the frequency of transgene specific CD4⁺ **(E)** and CD8⁺ **(F)** T-cells by IFN-γ ELISPOT. Dots represent individual mice. Horizontal bars indicate the average values. All statistical analyses are performed using Mann-Whitney test. *P < 0.05; **P < 0.005. IM, intramuscular; rAAV, recombinant adeno-associated viral vector; SGCAHY, human α-sarcoglycan gene fused to HY gene sequences encoding Uty and Dby male epitopes; CMV, Cytomegalovirus; TA, tibialis anterior; HE, Hematoxylin-Eosin.

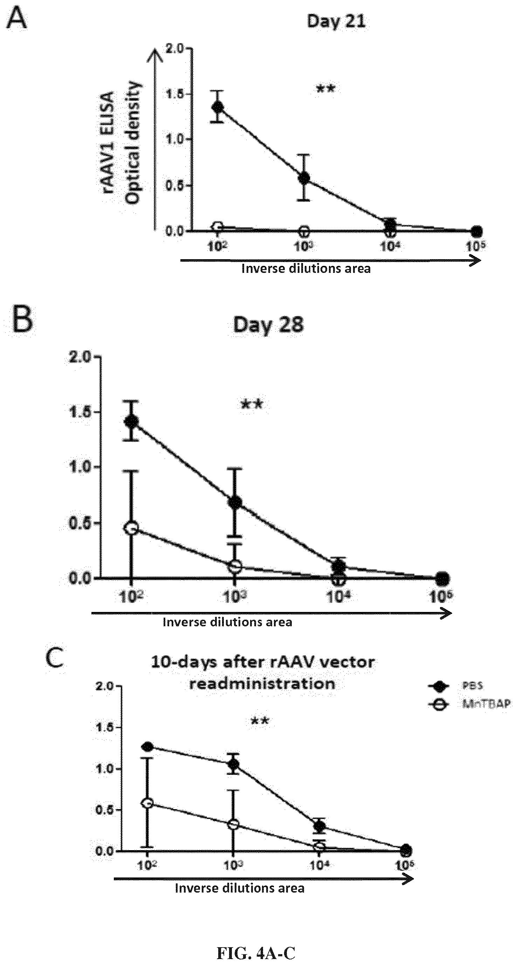

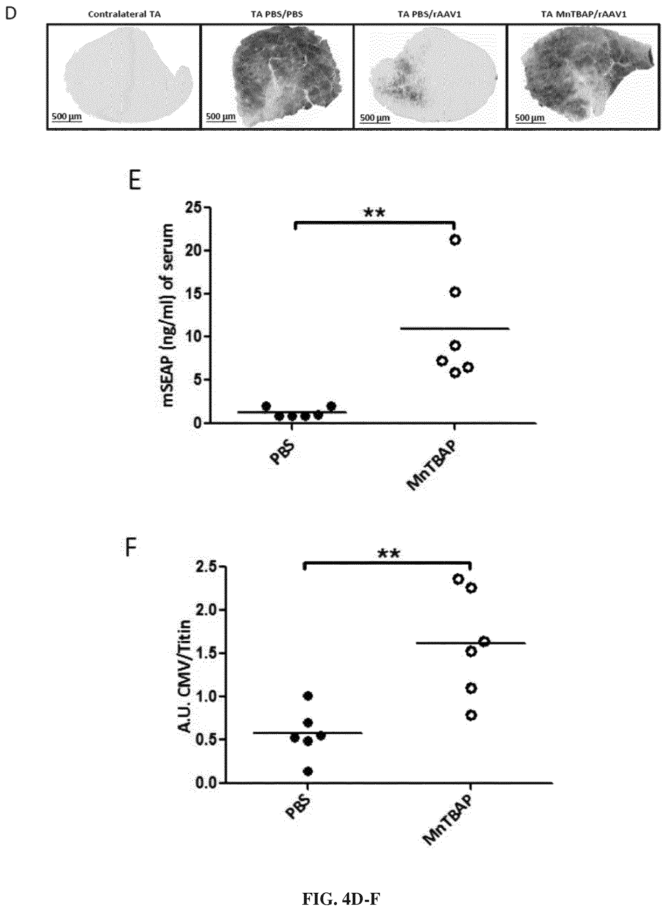

**Figure 4****:** MnTBAP inhibits primary and memory humoral response against capsid allowing efficient readministration of rAAV vector in muscle. C57BL/6 mice are IP multi-injected with MnTBAP (80 mg/kg daily during five days) or with an equivalent volume of PBS. Two hours post first injection of MnTBAP, mice are IV administrated with PBS or rAAV CMV SGCAHY vector (2.5.10⁹ vg per mouse). **(A** and **B)** Analysis of capsid-specific primary humoral response. Anti-rAAV1 IgG titers was measured by ELISA in serum samples harvested at D21 **(A)** and D28 **(B)** post vector injection. Curves represent the mean ± SD of optical density (OD) obtained at the indicated dilutions of sera from 6 mice per group and compare anti-rAAV IgG response in MnTBAP treated mice (closed circles) and PBS treated mice (open circles). **(C)** Analysis of capsid-specific memory humoral response after rAAV readministration in TA. Each mouse was subjected to a second injection of rAAV (2.10⁹ vg per mouse of rAAV1 CMV mSeap vector, AAV serotype 1 encoding the mSeap reporter gene,) into the left TA, 28 days post first injection of vector. Measurement of anti-rAAV 11gG titers by ELISA was performed in serum samples harvested 10 days after rAAV vector readministration. For each panel, statistical analyses with Mann-Whitney test are performed on area under the curve (AUC) values, obtained from ODs at four dilution points for each mouse serum. **(D)** Histochemical detection of mSeap in injected TA harvested 93 days after vector readministration. In first panel, non-injected right contralateral TA is represented as negative control. Second panel shows injected left TA with the rAAV1 CMV mSeap vector alone as positive control of mSeap expression in muscle. Third and fourth panels show administered left TA with the rAAV1 CMV mSeap vector in PBS or MnTBAP treated mice, respectively, and who received an initial systemic injection of rAAV1CMV SGCAHY vector. Images are representative of one mouse out of 6 per group. Scale bar = 500 µm. **(E)** Serum level of mSeap measured by a chemiluminescent assay from mice harvested 93 days after vector readministration. Dots represent individual mice (closed and open circles show PBS and MnTBAP treated mice, respectively). Statistical analyses are performed using Mann-Whitney test. **(F)** Copy number analysis of readministered rAAV1 in injected TA by quantitative CMV PCR assay with endogeneous titin gene for normalization. Data are expressed in arbitrary units (AU) relative to titin DNA. Each symbol represents a mouse (closed and open circles show PBS and MnTBAP treated mice, respectively). Statistical analyses are performed using Mann-Whitney test. **P< 0.005.

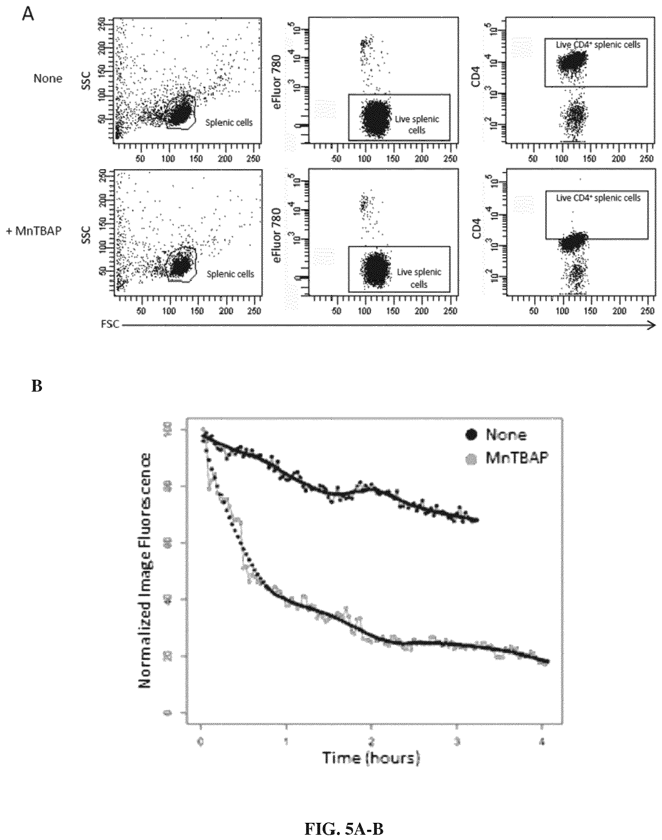

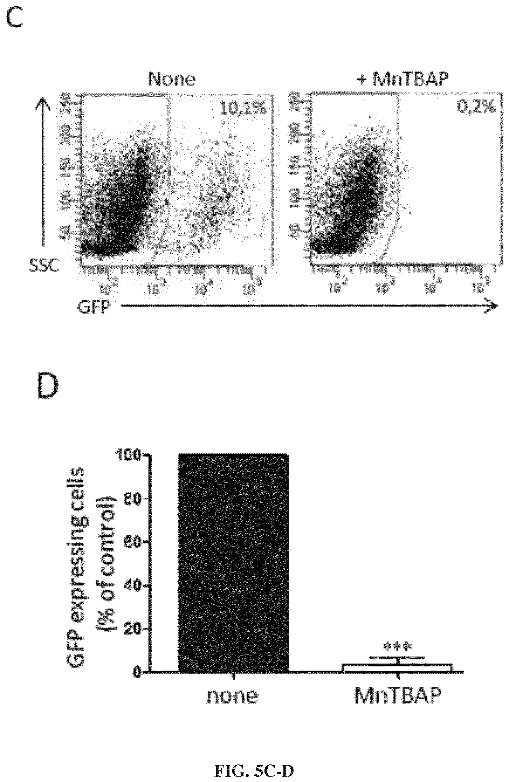

**Figure 5****:** MnTBAP induces rapid disappearance of CD4 at the cell surface of CD4 expressing cells. **(A)** Flow cytometry analysis of splenic enriched CD4⁺ T cells after treatment in complete medium for 2 hours with or without MnTBAP at 400 µM. Dot plots show gated splenic cells (left panels) according to Forward Scatter (FSC)/Side Scatter (SSC) parameters; live splenic cells (middle panels) according to FSC and the viability dye, eFluor 780; and live CD4⁺ splenic cells (right panels) according to FSC and CD4 expression. One representative experiment out of at least 6 is shown **(B)** Time lapse analysis of CD4 cell surface over-time decline on CD4⁺ T cells. After CFSE and CD4 staining, splenic enriched CD4⁺ T cells are incubated in the presence or absence of MnTBAP (400 µM) and subjected to live cell time lapse image acquisition every 120 sec for at least 4 h. CD4 fluorescence intensities, normalized to the maximum fluorescence measured at t0, are reported for each condition. One representative experiment out of 2 is shown **(C).** MnTBAP-induced loss of CD4 on the surface of human T-cells inhibits their transduction by lentiviral vectors pseudotyped with HIV-1 envelope protein (HXB2-env). CD3⁺ T cells from human peripheral blood are transduced in the presence or absence of MnTBAP (400 µM) on retronectin coated-plates, with HXB2-env pseudotyped lentiviral vectors expressing GFP reporter gene. Transduction efficiency was assessed by flow cytometry via GFP expression 72 hours post transduction. Representative dot plots are shown with inset numbers indicating the percentage of transduced GFP⁺ cells. **(D)** Normalized percentages of GFP⁺ cells obtained 72 hours post transduction. The percentage of GFP⁺ cells in the cultures without MnTBAP (none) is set to 100%. (n=3 independents experiments). Statistical analysis was with the paired t-test. ***P < 0.0001.

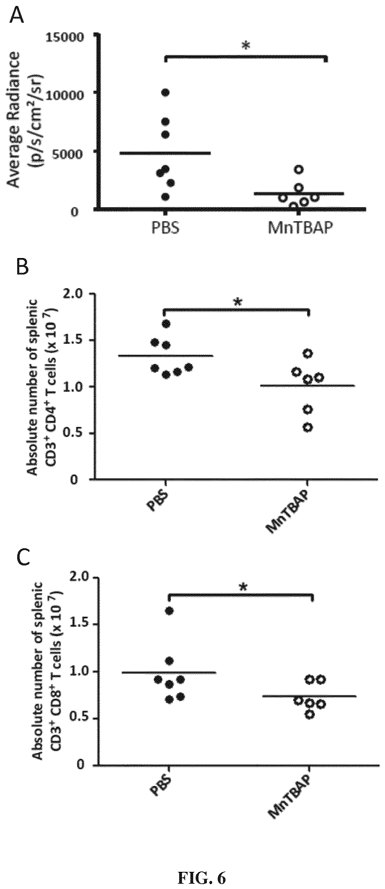

**Figure 6****:** MnTBAP reduces inflammation induced by AAV-mediated gene transfer, and the absolute number of splenic T-lymphocyte subsets. Mice are, daily during five days, IP injected with MnTBAP or with an equivalent volume of PBS. Two hours post first injection of MnTBAP, mice are administrated with rAAV1 CMV SGCAHY vector in the left TA. **(A)** Quantification of the average radiance of a Region Of Interest (ROI) drawn around the left inferior limb of each mouse. PBS-treated mice exhibit a significantly higher average radiance therefore inflammation level than MnTBAP-treated mice. (n=2, 3-4 mice per group). **(B** and **C)** Decreased absolute numbers of splenic T-lymphocyte subsets 15 days post first injection of MnTBAP. The absolute numbers of CD4⁺ **(B)** and CD8⁺ **(C)** T cells in spleen are analyzed by flow cytometry by gating on the CD4⁺ or CD8⁺ population among the living CD3⁺ population. (n=2, 3-4 mice per group). All statistical analyses are performed using Mann-Whitney test. *P < 0.05.

### EXAMPLES

The present invention is further illustrated by the following examples.

### MATERIALS AND METHODS

### Animals

Six- to eight-week-old female C57BL/6 mice are purchased from Charles River Laboratories. Mice are housed under specific pathogen-free conditions and handled in accordance with French and European directives. Protocols are conducted according to national and international ethical standards and are approved by the local ethics committee.

### In vitro and in vivo MnTBAP treatments

MnTBAP (Calbiochem, San Diego, USA or Enzo Life Sciences, Farmingdale, USA) is dissolved in 75 mM NaOH to constitute a 20 mM stock solution. MnTBAP is dissolved in the complete medium, used for *in vitro* treatment, containing RPMI-1640 supplemented with 10% heat-inactivated fetal calf serum, 100 U/ml penicillin/streptomycin, 2 mM glutamax and 50 µM β-mercaptoethanol β-ME). Hypertonic medium, used to block clathrin dependent endocytosis, is complete medium supplemented with 0.45 M sucrose. For *in vivo* treatment, the stock solution is diluted with PBS and administered at 80 mg/kg intraperitoneally daily during five days. Control animals are injected with equivalent volume of PBS.

### SplenicCD4⁺ T cells isolation and flow cytometry analysis

After mechanic disruption of the spleen, erythrocytes are eliminated by hypotonic shock with ammonium chloride potassium (ACK) lysing buffer. Then CD4⁺ T cells are isolated from spleen cell suspension by negative selection using a CD4⁺ T cell isolation kit (Miltenyi Biotec, Paris, France) followed by auto-MACS (Miltenyi Biotec) magnetic sorting, according to the manufacturer's instructions. Purity of isolated CD4⁺ T cells is usually > 95%. Cell suspensions are first incubated with anti-FcγRIII/II (Fc Block, 2.4G2, BD Biosciences, Le Pont de Claix, France) monoclonal antibodies (mAb) for 10 minutes at 4°C and then stained for 30 minutes at 4°C in PBS, 0.1% bovine serum albumin (BSA) with Fc Block present, using saturating amounts of rat anti-mouse CD4-Pacific Blue (RM4-5, BD Biosciences) mAb. Gates for CD4⁺ populations are set according to isotype controls. Cell viability is determined by staining with 7-aminoactinomycin D (7-AAD; Sigma-Aldrich, St-Quentin-Fallavier, France) or Fixable Viability Dye eFluor 780 (eBiosciences, Paris, France). When indicated, samples are fixed with 1% paraformaldehyde. Samples are acquired on an LSR II flow cytometer (BD Biosciences) and data were analyzed with DIVA software (BD Biosciences).

### CD4 Immunoprecipitation

Cells, harvested after 2 hours of culture with or without MnTBAP, are resuspended in solubilization buffer containing 1% NP-40 (Sigma-Aldrich), 0.1 M (NH₄)₂SO₄, 20 mM Tris (pH 7.5), 10% glycerol, 25 mM NaF (Sigma-Aldrich), 1 mM Na₃VO₄ (Thermo Fisher Scientific, Villebon-sur-Yvette, France) and 1x protease inhibitor mixture (Roche, Basel, Switzerland). After 30 minutes of incubation under gentle agitation, cell lysates are centrifuged at 14,000 x g for 30 minutes. Lysates are frozen in aliquots at -20°C or used immediately. Immunoprecipitation is performed by incubation overnight of lysates with rat anti-mouse CD4 (GK 1.5, Biolegend, San Diego, CA, USA) under gentle agitation at 4°C. The antigen-antibody complex is then added to Protein A/G resin slurry (Pierce, Thermo Fisher scientific), and incubated with gentle mixing for 2 hours at room temperature, and finally microcentrifuged for 30 seconds. The pellet is washed five times with solubilization buffer, and the immunoprecipitated material is eluted by incubating the complex for 2-5 minutes at 95-100°C in Laemmli sample buffer prepared without β-ME. The immunoprecipitates and whole-cell lysates ("Input") are electrophoresed in precast SDS-PAGE (10% polyacrylamide) gels (Bio-Rad Laboratories, Hercules, CA, USA) transferred to PVDF membrane (Merck, Darmstadt, Germany), and probed with anti-p56^{Lck} rabbit antibody (Santa Cruz Biotechnology, Nanterre, France) followed by IRDye conjugated secondary antibody according to the manufacturer's instructions (LI-COR Biosciences, Lincoln, NE). Infrared fluorescence of the secondary antibodies was read on an Odyssey Imaging System, and quantification of bands was performed using the ImageStudio Lite software (LI-COR Biosciences).

### Immunofluorescence microscopy

After culture in complete medium with or without MnTBAP at 400 µM, murine splenic CD4⁺ T cells are collected and subjected to immunostaining as previously described (T. M. Neildez-Nguyen et al., Immunology 144, 431. 2015). Briefly, after adhesion on poly-L-lysine-coated slides (Thermo Fisher Scientific), cells are fixed in 4% paraformaldehyde for 5 minutes at 4°C and for 10 minutes at room temperature, and permeabilized with methanol for 6 minutes at -20°C. Cells are stained overnight with rat anti-mouse CD4-APC (BD Biosciences) and rabbit anti-mouse clathrin (Abcam, Paris, France) antibodies, and then with the goat anti-rabbit Alexa Fluor 488 secondary antibody (Invitrogen, Carlsbad, USA) for 30 minutes at room temperature. Negative controls are performed with the corresponding isotype controls of primary antibodies used (BD Biosciences and Abcam). The slides are mounted with Fluoromount/Dapi (SouthernBiotech, Montrouge, France). Images are acquired with confocal-laser-scanning microscope (Leica Microsystems, Nanterre, France).

### rAAV vector production

Recombinant non-replicative, adenovirus-free rAAV2/1_CMV_SGCA_HY and rAAV2/1_CMV_mSeAP vector preparations are generated by transient transfection of HEK293 cells with three endotoxin-free plasmids (Macherey-Nagel, Hoerdt, France): (1) the plasmid packaging (pLTR CO2) suppling the AAV2 rep and cap genes of AAVI encoding the serotype 1 capsid, (2) the plasmid helper (pXX6) providing the adenovirus helper functions and (3) the plasmid encoding the transgene. For rAAV2/1_CMV_SGCA_HY vector, a chimeric transgene (pGG1 SGCA HY) is used, consisting of the human alpha-sarcoglycan (SGCA) gene fused to sequences encoding T cell epitopes of the murine HY gene (Dby peptide (SEQ ID NO: 1; NAGFNSNRANSSRSS) presented by I-Ab and the Uty peptide (SEQ ID NO: 2; WMHHNMDLI) presented by H2-Db) and expressed from the cytomegalovirus (CMV) promoter (F. Boisgerault et al., Human gene therapy 24, 393. 2013). For rAAV2/1_CMV_mSeAP vector, pGG2-CMV-muSeAP plasmid encoding the secreted form of the murine alkaline phosphatase under the control of the CMV-IE promoter is used (C. Riviere et al., Gene therapy 13, 1300. 2006). rAAV2/1 vectors are purified by centrifugation over a CsCl gradient, and tittered in viral genome (vg) by real-time PCR as previously described (C. Riviere et al., Gene therapy 13, 1300. 2006). Vector final suspension is tested for endotoxin level and found to be < 10 EU/ml.

### OVA peptides subcutaneous immunization

A mixture of chicken egg albumin (OVA) peptides is administrated subcutaneously in mice anesthetized by intraperitoneal injection of xylazine (10 mg/kg) and ketamine (100 mg/kg). OVA257-264 (SEQ ID NO: 3; SIINFEKL) (InvivoGen, San Diego, USA) is a class I (Kb)-restricted peptide epitope of OVA presented by the class I major histocompatibility complex molecule (CMH) H-2Kb. OVA323-339 (SEQ ID NO: 4; ISQAVHAAHAEINEAGR) (AnaSpec, San Jose, USA) is an H-2b-restricted OVA class II epitope. 100 µg of each peptide complexed with the same volume of incomplete Freund's adjuvant (IFA) (Sigma-Aldrich) are administrated. Controls are injected with PBS-IFA complexes. OVA peptides- and PBS-IFA complexes are injected two hours or 21 days post first intraperitoneal injection of MnTBAP (or PBS). Eight days post OVA peptides injection, mice are sacrificed and spleens harvested to analyze OVA-specific T cell response.

### rAAV vector delivery

*Intramuscular (IM) injection:* Two hours post first intraperitoneal (IP) injection of MnTBAP, mice are anesthetized by IP injection of xylazine (10 mg/kg) and ketamine (100 mg/kg) and 25 µl of a rAAV2/1 CMV SGCA HY viral preparation containing 2.5.10⁹ vg is injected in the left tibialis anterior muscle (TA). Controls are injected with PBS. Mice are sacrificed 15 days after vector injection and the injected muscles are harvested to perform histologic analysis. Spleens are harvested to analyze transgene-specific T cell response.

*Intravenous (IV) injection:* Two hours post first IP injection of MnTBAP, mice are anesthetized by IP injection of xylazine (10 mg/kg) and ketamine (100 mg/kg) and 200 µl of a rAAV2/1 CMV SGCA HY viral preparation containing 2.5.10⁹ vg is injected into retro orbital sinus or into tail vein. Controls are injected with PBS. Serum samples are harvested at D21 and D28 post vector injection to analyze capsid-specific humoral response.

*Readministration protocol:* After the initial IV injection of rAAV2/1 CMV SGCA HY vector at D0 (two hours post first IP injection of MnTBAP) the second rAAV1 injection (2.10⁹ vg of rAAV2/1 CMV mSeAP) is performed 28 days later in the left TA muscle. Mice are followed up to 93 days after readministration to analyze capsid-specific humoral response and to measure circulating mSeAP from serum samples. Mice are killed 93 days after second injection of vector. Left and right TA muscles are harvested for histological and enzymatic assay.

### Histologic analysis

Mice are killed at indicated time points. Injected muscles are harvested, snap frozen in isopentane previously chilled in liquid nitrogen and kept at -80°C until processing. Transverse cryosections (8 µm thick) of the muscles are either stained with hematoxylin-eosin (HE) to evaluate inflammation and muscle integrity or used for colorimetric immunostaining or for mSeAP detection.

For colorimetric immunodetection of CD8a, cryosections are rehydrated with PBS for 5 min and then incubated 20 min with H2O2 to inhibit endogenous peroxidases at room temperature (RT). After washing with PBS, sections are blocked with PBS, 10% goat serum for 30 minutes and then incubated with 1/40 dilution of a rat monoclonal primary antibody directed against CD8a (Thermo Fisher scientific), 1-2 hours at RT. After washing with PBS, sections are incubated with secondary antibody goat anti-rat conjugated with horseradish peroxidase (HRP) (Invitrogen) diluted 1/600 for 1hour at RT. Sections are washed three times with PBS and then incubated with diluted diaminobenzidine (DAKO, Trappes, France) for 2-5 minutes. Then, sections are successively treated with ethanol (5 minutes), twice in xylene (5 minutes) and mounted with Eukkit (Labonord, VWR International, Fontenay-sous-Bois, France). The slides are examined with a Nikon microscope (Champigny sur Marne, France). Digital images are captured using CCD camera (Sony) and processed using Cartograph 5.5 (Microvision Software; Microvision, Evry, France).

For mSeAP histochemical detection, transverse cryosections of TA muscles are fixed in 0.5% glutaraldehyde and washed twice with PBS. Endogenous alkaline phosphatase is heat-inactivated for 30 minutes at 65°C. Sections are then incubated for 5 hours at 37°C in 0.165 mg/ml 5-bromo-4-chloro-3-indolyl-phosphate and 0.33 mg/ml of nitroblue tetrazolium in 100 mM Tris-HCl, 100 mM NaCl and 50 mM MgCl₂. Sections are counterstained with nuclear fast red.

### Measure of transgene and CD8 expression by real-time reverse transcriptase-PCR (RT-PCR)

Total RNA is extracted from muscles using Trizol (Invitrogen). Residual DNA is removed from the samples using the Free DNA removal kit (Ambion, Courtaboeuf, France). CDNA is synthesized from 1 µg of RNA using a mix of random hexamers and anchored oligo dT 3:1 according to the protocol Thermo Scientific Verso cDNA Synthesis kit (Thermo Fisher Scientific). Real-time PCR is performed with 0.2 µM of each primer and 0.1 µM of the probe according to the protocol Thermo Scientific Absolute qPCR ROX Mix. The primer pairs and Taqman probes used for ha-sarcoglycan amplification are: 920hasarco.F: 5'-TGCTGGCCTATGTCATGTGC-3' (SEQ ID NO: 5), 991hasarco.R: 5'TCTGGATGTCGGAGGTAGCC-3' (SEQ ID NO: 6), and 946hasarco.P: 5'-CGGGAGGGAAGGCTGAAGAGAGACC-3' (SEQ ID NO: 7). The ubiquitous acidic ribosomal phosphoprotein gene (PO) is used to normalize the data across samples. The primer pairs and Taqman probe used for PO mRNA amplification are: m181PO.F: 5'-CTCCAAGCAGATGCAGCAGA-3' (SEQ ID NO: 8), m267PO.R: 5'-ACCATGATGCgCAAGGCCAT-3' (SEQ ID NO: 9), and m225PO.P: 5'-CCGTGGTGCTGATGGGCAAGAA-3' (SEQ ID NO: 10). The primer pairs and Taqman probes for the mouse CD8a are Mm01182107₋g1 (Thermo Fisher Scientific).

### Genomic DNA extraction and real-time quantitative-PCR

Genomic DNA is extracted from frozen muscles using MagNA Pure 96 Instrument (Roche). Real-time PCR to amplify CMV in relation to the titin gene is performed using ABI PRISM 7900 system (Life Technology, Saint-Aubin, France) with 0.1 µM of each primer and 0.1 mM of the probe according to the protocol Absolute QPCR Rox Mix (ABgene, Cambridge, UK). The primer pairs and Taqman probes used for CMV and titin gene amplification are: mTitin-F: 5'-AAAACGAGCAGTGACGTGAGC-3' (SEQ ID NO: 11), mTitin-R: 5'-TTCAGTCATGCTGCTAGCGC-3' (SEQ ID NO: 12), mTitin-P: 5'-TGCACGGAAGCGTCTCGTCTCAGTC-3' (SEQ ID NO: 13), mCMV-F: 5'-CATCAATGGGCGTGGATAGC-3' (SEQ ID NO: 14), mCMV-R: 5'-GGAGTTGTTACGACATTTTGGAAA-3' (SEQ ID NO: 15), mCMV-P: 5'-ATTTCCAAGTCTCCACCC-3' (SEQ ID NO: 16). Results are calculated from a standard curve based on dilutions of 2 plasmids containing either the Titin or CMV sequences. Background levels are comprised between 36-40 CT values.

### IFN-γ enzyme-linked immunospot assay (IFN-γ ELISPOT)

Spleens are harvested to analyze the cellular immune anti-transgene response by T-cell cytokine response assay. Multiscreen-HA Assay System 96-well Filtration plates (Merck) are coated overnight with 2.5 µg/ml of anti-mouse IFN-γ purified mAb (eBiosciences) at 4°C, washed, and then blocked with RPMI 1640 medium (Life Technologies, Saint-Aubin, France) supplemented with 10% heat inactivated fetal bovine serum (FCS), 100U/ml penicillin/streptomycin (Thermo Fisher Scientific) and 2mM GlutaMAXTM-I (Life technologies) for 2 hours at 37°C. Plates are washed three times with RPMI 1640 medium without FCS. Splenocytes are added in duplicate to the IFN-γ-coated well (1 or 0.5 x 10⁶ cells/well) and cultured in presence of Dby or Uty peptide (Genepep, Montpellier, France) at 2 µM or in presence of OVA peptides at 10 µg/ml. As a positive control, cells are stimulated with Concanavalin A (Sigma-Aldrich) at 1 µg/ml in each test. After 24 hours, plates are washed three times with PBS, 0.05% Tween 20 and three times with PBS alone before 2 hours incubation at RT with 1 µg/ml biotinylated anti-mouse IFN-γ mAb in PBS/0.1% BSA. Plates are washed three times with PBS/0.05% Tween 20 and three times with PBS alone before 1 hour 30 incubation with 0.66U/ml streptavidin-alkaline phosphatase (AP) conjugate (Roche Diagnostics GmbH, Mannheim, Germany) at room temperature. After wash step with PBS/0.05% Tween 20, and with PBS alone, spots are developed using BCIP/NBT-plus (Mabtech AB, Nacka Strand, Sweden). Spots are counted using an AID reader (Ceipheid Benelux, Louven, Belgium). Spot forming units (SFU) per million cells for each mouse are calculated as the average value of duplicate measures after subtraction of background values obtained with *in vitro* unstimulated splenocytes.

### Measurement of anti-AAVl antibody titers

rAAVl-specific serum antibody titers are evaluated by ELISA. F96 Maxisorp Nunc Immuno plates (Thermo Fisher Scientific) are coated overnight at 4°C with 0.2 x 10⁹ vg of AAV1 vector diluted in 0.1M carbonate coating buffer (pH 9.6). Plates are washed three times and blocked with a solution of 6% milk in PBS for 2 hours at room temperature. Serial dilutions of samples in this blocking buffer (10-fold dilutions starting 10⁻² to 10⁻⁵ are tested) are incubated on the plates for 1 hour at 37°C. Plates are washed three times, incubated with a horseradish peroxidase-conjugated goat antibody directed against mouse IgG (Southern Biotech, Birmingham, AL) and revealed by TMB Substrate reagent Set (BD Biosciences). Reaction is stopped with an H₂SO₄ solution and the optical density at 450 nm is determined using a luminometer (Discovery HT-R, BioServ, Thiais, France) with KC4 software. The area under the curve (AUC) values obtained from the optical densities at four dilution points are calculated for statistical analysis using GraphPad Prism software (Logi Labo, Paris, France).

### Quantification of circulating mSeAP

Determination of alkaline phosphatase concentration in immunized mice sera is performed by chemoluminescence analyzis (C. Riviere et al., Gene therapy 13, 1300. 2006). Blood samples are centrifuged for 10 min at 8000 rpm to collect sera, endogenous alkaline phosphatase is heat inactivated for 5 min at 65°C and the heat-resistant mSeAP is measured by addition of the reaction buffer and CSPD® chemiluminescent substrate, according to the manufacturer's instructions (Tropix, Applied Biosystems, Forster City, USA). Chemiluminescence is quantified using a luminometer, Enspire (Perkin Elmer, Massachusetts, USA). Expression levels are expressed as ng of mSeAP per ml of serum using a standard curve of purified human placental alkaline phosphatase.

### Statistics

Statistical analyses are performed using GraphPad prism softaware. For antibody production, differences are analyzed by comparing the average values of the area-under-the-curve for the titration of each mouse serum. Statistical significance of the difference between groups is determined using Mann-Whitney test. Significant differences are defined with probability values inferior to 0.05 (*P < 0.05, **P< 0.005, ***P< 0.001).

### Time-lapse microscopy

Murine splenic CD4⁺ T cells are first stained with CFSE, according to the manufacturer's recommendations (Invitrogen), and then with rat anti-mouse CD4-APC (RM4-5, BD Biosciences) mAb. After staining, cells are incubated in Ibidi's µ-chambers culture dish (Ibidi, Nanterre, France) in the thermo-regulated chamber of the Zeiss Axiovert 100 M confocal microscope. A time lapse acquisition is performed with a delay of 120 sec between frames. Each frame is illuminated using a 488 nm and 633 nm lasers, and fluorescence collected using 505-580 nm and 644-719 nm filters. The magnification is 40x/1.3 oil and images are saved as 8bits. The image stack is splitted using ImageJ-1.43, and images analyzed using CellProfiler 2.0.10415. The cell segmentation is performed on the CFSE fluorescence, and the fluorescence of the CD4 staining analyzed using the R software. Intensity is normalized to the maximum fluorescence measured at T0 for each condition.

### Culture and Transduction of Human Primary Cells

CD3⁺ T cells, from peripheral blood of healthy individual donors, are purified by positive selection via MACS beads using a magnetic cell sorting system (Miltenyi Biotec). CD3⁺ T cells are activated for 3 days at 37°C, 5% CO2 in plates coated with anti-CD3 (10 µg/ml) and anti-CD28 (10 µg/ml) antibodies (both from Miltenyi Biotec) and filled with RPMI 1640 medium supplemented with 10% heat-inactivated fetal calf serum, 100 U/ml penicillin/streptomycin, 2 mM glutamax, 100 IU/ml IL-2 (Miltenyi Biotec). Cells are then transferred on retronectin (20 µg/cm2) coated-plates, in the presence or absence of MnTBAP (400 µM), at a density of 2.5 x 10⁵ cells/ml for transduction during 6 h with 6 x 10⁷ physical particles (pp)/ml of HXB2-env pseudotyped lentiviral vectors expressing GFP reporter gene under the control of hPGK (human phosphoglycerate kinase 1). After transduction, cells are washed and further incubated for 72 h in fresh culture medium (RPMI 1640 medium supplemented as described above). Cells are then harvested for transduction efficiency analysis by FACS, determined as the percentage of viable (7AAD-) lymphoid cells expressing GFP⁺. The data are normalized to be a percentage of the control value (none) from each experiment.

### In vivo chemiluminescence imaging of localized inflammation

After mice isofluorane anaesthesia, 100 µl of L-012 solution (15 mg/ml) (Wako, Osaka, Japan) are administered intravenously in retro-orbital sinus 4 days after first MnTBAP injection. 2 minutes after L-012 administration, mice are placed in an *in vivo* imaging system (IVIS Lumina, Perkin Elmer, USA), and chemiluminescence images are acquired for each mouse with a typical exposure time of 5 minutes. Images are quantified using LivingImage (Perkin Elmer, USA), by drawing a Region of Interest around the left inferior limb of the mouse. Radiance (ph/s/cm²/sr) is measured for each Region of Interest (ROI).

### Spleen cells staining and flow cytometric analysis

After mechanic disruption of the spleen, erythrocytes are eliminated by hypotonic shock with ammonium chloride potassium (ACK) lysing buffer. Cell suspensions are first incubated with anti-FcγRIII/II (clone 2.4G2, BD Biosciences, San Jose, USA) monoclonal antibodies (mAb) for 10 minutes at 4°C and then stained for 30 minutes at 4°C in PBS with 0.1% bovine serum albumin (BSA) using saturating amounts of the following mAbs: rat anti-mouse CD4-Pacific Blue, hamster anti-mouse CD3-APC (145-2C11, BD Biosciences), rat anti-mouse CD8-Alexa fluor 700 (53-6.7, eBiosciences). Dead cells are excluded using 7-amino-actinomycin D (7AAD) (Sigma-Aldrich). All FACS data are collected on a LSRII flow cytometer using the Diva software (BD Biosciences).

### RESULTS

### Example 1: MnTBAP-induced reversible CD4 internalization mechanism

A progressive decrease of CD4 expression is observed on viable murine splenic enriched CD4⁺ T cells treated for 2 hours with increasing doses of MnTBAP (0, 100, 200, 400 µM). A maximal decrease of CD4 expression, leading to the almost complete disappearance of the T cell population, is observed with the highest dose of MnTBAP (400 µ,M) **(****Figures 1A** and **1B****).**

According to these results showing a dose-effect of MnTBAP on CD4 expression, it is decided to focus on the concentration of 400 µM of MnTBAP for the next experiments.

A disruption of CD4 and P56^{Lck} is observed on the surface of viable murine splenic enriched CD4⁺ T cells treated for 2 hours with MnTBAP **(****Figures 1C** and **1D****).**

A colocalization of clathrin and CD4 is observed when viable murine splenic enriched CD4⁺ T cells are treated for 2 hours with MnTBAP (right panel, indicated by the arrows), compared to a non-treated condition (left panel) **(Figure IE).**

Under normal tonicity conditions, the percentage of CD4 expression on murine splenic enriched CD4⁺ T cells is decreased after treatment with MnTBAP compared to the non-treated condition. Under hypertonic conditions, known to inhibit specifically clathrin-dependent endocytosis, no significant difference of CD4 expression is observed on murine splenic enriched CD4⁺ T cells between the treated and the non-treated conditions **(****Figure 1F****).**

Thus, the CD4 internalization is dependent on a clathrin mediated-endocytosis mechanism.

A decrease of CD4 expression on murine fixed splenic enriched CD4⁺ T cells treated 2 hours with MnTBAP ("treated") is observed compared to the non-treated condition ("none"). Two hours after washing the "treated" cells to remove MnTBAP from the cell culture (condition "2 h removed"), the decrease of CD4 expression is less important compared to the "treated" condition. The decrease of CD4 expression is further less accentuated when the "treated" cells stay overnight ("O/N removed" condition) after removing MnTBAP from the cell culture **(****Figure 1G****).**

Thus, MnTBAP induces a reversible CD4 internalization from the surface of murine splenic enriched CD4⁺ T cells.

### Example 2: MnTBAP inhibits in vivo temporarily specific T-cell immune responses against OVA peptides

Enriched splenic CD4⁺ T cells harvested from mice immunized with OVA peptides two hours after the first MnTBAP injection (condition "MnTBAP + OVA / IFA") are not able to produce IFN-γ compared to splenic enriched CD4⁺ T cells harvested from immunized non-treated mice ("PBS + OVA / IFA" condition). No IFN-γ production is observed in the corresponding control conditions ("PBS + PBS / IFA" condition and "MnTBAP + PBS / IFA" condition) (**Figure 2A****,** left panel).

Splenic enriched CD8⁺ T cells harvested from mice immunized two hours after the first MnTBAP injection ("MnTBAP + OVA / IFA" condition) are not able to produce IFN-γ compared to the "PBS + OVA / IFA" condition. No IFN-γ production is observed in the corresponding control conditions (**Figure 2A****,** right panel).

Splenic enriched CD4⁺ T cells harvested from mice immunized with OVA peptides 21 days after the first MnTBAP injection (``MnTBAP + OVA / IFA" condition) produce low levels of IFN-γ with no significant differences compared to the "PBS + OVA / IFA" condition. No IFN-γ production is observed in the corresponding control conditions (**Figure 2B****,** left panel).

Splenic enriched CD8⁺ T cells harvested from mice immunized with OVA peptides 21 days after the first MnTBAP injection ("MnTBAP + OVA / IFA" condition) produce IFN-γ with no significant differences compared to the "PBS + OVA / IFA" condition. No IFN-γ production is observed in the corresponding control conditions (**Figure 2B****,** right panel).

Therefore, MnTBAP inhibits temporarily CD4 and CD8 T-cell responses in mice immunized with OVA peptides.

### Example 3: MnTBAP prevents muscle destruction, CD8⁺ T cell infiltrate and prolongs transgene expression by decreasing transgene-specific CD8⁺ and CD4⁺ T-cell responses induced by IM rAAV gene transfer

Fifteen days post vector injection, mice pretreated with MnTBAP ("MnTBAP/rAAV1" condition) have a normal muscle integrity **(****Figures 3A** and **3B****),** a decrease of CD8a mRNA expression **(****Figure 3C****)** and an increase of the SGCA transgene expression **(****Figure 3D****)** compared to non-treated control groups ("PBS/rAAV1" or "PBS/PBS" conditions).

Eight days post vector injection, the IFN-γ response of transgene specific CD4⁺ T cells harvested from MnTBAP treated mice is significantly decreased compared to transgene specific CD4⁺ T cells from mice that received PBS (**Figure 3E****,** upper panel). The same result is observed fifteen days post vector injection (**Figure 3E****,** lower panel).

Eight days post vector injection, no significant differences is observed from transgene specific CD8⁺ T cells in the two conditions (**Figure 3F****,** upper panel). However, fifteen days post vector injection, the IFN-γ response from the transgene specific CD8⁺ T cells harvested from treated mice is significantly decreased (**Figure 3F****,** lower panel).

MnTBAP is thus able to decrease the transgene-specific CD8⁺ and CD4⁺ T-cell responses (i.e. IFN-γ response) induced by an intramuscular rAAV gene transfer.

### Example 4: MnTBAP inhibits primary and memory humoral responses against capsid allowing an efficient readministration of rAAV vector in the muscle

Day 21 and Day 28 post vector injection, mice pretreated with MnTBAP produce no or significantly less specific anti-rAAV IgG compared to the control group **(****Figures 4A** and **4B****).** A vector readministration 28 days post vector first injection leads to a decrease of specific anti-rAAV IgG titer compared to the control group that received PBS **(****Figure 4C****).**

A detection of the reporter gene mSeap (murine secreted alkaline phosphatase) is observed in the muscle harvested from mice pretreated with MnTBAP and readministered with the vector **(****Figure 4D****).**

The level of mSeap in the serum of mice pretreated with MnTBAP and readministered with the vector is significantly increased **(****Figure 4E****)** compared to control mice. The normalized copy number of the readministered rAAV is significantly increased compared to control mice **(****Figure 4F****).**

Therefore, MnTBAP inhibits primary and memory humoral response against the vector.

### Example 5: MnTBAP induces rapid disappearance of CD4 at the cell surface of CD4 expressing cells

MnTBAP induces a disappearance of CD4 at the cell surface of CD4 expressing cells compared to the control condition. This effect was not due to the induction of cell death as MnTBAP treatment doesn't result in more eFluor 780+ dead cells, while leading to the disappearance of the genuine CD4⁺ T lymphocyte population **(****Figure 5A****).**

A time lapse analysis for 4 hours of CD4 cell surface shows that CD4 declines on CD4⁺ T cells in the presence of MnTBAP compared to the control condition **(****Figure 5B****).**

The transduction efficiency of human T cells by lentiviral vectors pseudotyped with HIV-1 envelope protein is significantly decreased after MnTBAP treatment compared to the control condition **(****Figures 5C and 5D****).**

### Example 6: MnTBAP reduces inflammation induced by AAV-mediated gene transfer, and the absolute number of splenic T-lymphocyte subsets

Mice that received MnTBAP produce less Reactive Oxygen Species (ROS) than mice that received PBS after gene transfer **(****Figures 6A****).** In addition, mice that received MnTBAP have a significant decrease in the absolute number of splenic CD4⁺ and CD8⁺ T cells compared to mice that received PBS after gene transfer **(****Figures 6B** and **6C****).**

Therefore, MnTBAP reduces CD4 and CD8 T cell immune responses after gene transfer.

## Claims

1. MnTBAP or analogs thereof for use in inducing a transient immune tolerance to an antigen in a subject, wherein said immune tolerance is maintained for less than 2 months after the last administration of MnTBAP.

2. MnTBAP or analogs thereof for use according to claim **1,** wherein MnTBAP is administered during 1 to 5 days, preferably once a day during 3 or 4 days.

3. MnTBAP or analogs thereof for use according to claim **1** or claim **2,** wherein a dose of MnTBAP ranging from about 1 to about 1000 mg/kg, preferably from about 50 to about 100 mg/kg, more preferably of about 80 mg/kg is to be administered to the subject.

4. MnTBAP or analogs thereof for use according to any one of claims **1** to **3,** wherein MnTBAP induces the reversible internalization of the CD4 molecules at the T cell membrane, thereby resulting in a transient immune tolerance.

5. MnTBAP or analogs thereof for use according to any one of claims **1** to **4,** wherein the subject is a human subject.

6. MnTBAP or analogs thereof for use according to any one of claims **1** to **5,** wherein said antigen is recognized by T cells and induces an unwanted immune response.

7. MnTBAP or analogs thereof for use according to claim **6,** wherein said unwanted immune response is an autoimmune disease, graft rejection or graft versus host disease (GVHD).

8. MnTBAP or analogs thereof for use according to any one of claims **1** to **7,** wherein the subject is affected with an autoimmune disease, graft rejection or graft versus host disease (GVHD).

9. MnTBAP or analogs thereof for use according to claim **8,** wherein said autoimmune disease is selected from the group comprising type 1 diabetes and autoimmune arthritis.

10. MnTBAP or analogs thereof for use according to any one of claims **1** to **9,** wherein the subject is an autoimmune disease patient exhibiting a flare up of said autoimmune disease.

11. MnTBAP or analogs thereof for use according to any one of claims **1** to **10,** wherein the subject is treated by gene therapy.

12. MnTBAP or analogs thereof for use according to claim **11,** wherein the antigen is the vector or the transgene or the transgene product used in gene therapy.

13. MnTBAP or analogs thereof for use in preventing or treating HIV in a subject, wherein MnTBAP prevents contamination of non-infected CD4- T cells by HIV particles.

14. MnTBAP or analogs thereof for use according to claim **13,** wherein the subject is a HIV patient, and wherein MnTBAP is administered in combination with a HIV treating agent.

15. MnTBAP or analogs thereof for use according to claim **13,** wherein the subject is or will be in contact with the HIV virus, and wherein MnTBAP administration prevents contamination of the subject by HIV virus.


SEQUENCE LISTING

<110> PSL - PARIS SCIENCES & LETTRES
      ÉCOLE PRATIQUE DES HAUTES ÉTUDES
      INSTITUT NATIONAL DE LA SANTÉ ET DE LA RECHERCHE MÉDICALE
      GÉNÉTHON
      NEILDEZ Thi My Anh

<120> MNTBAP OR ANALOGS THEREOF FOR USE IN INDUCING A TRANSIENT IMMUNE
TOLERANCE

<130> CV-736/EP

<160> 16

<170> BiSSAP 1.3.6

<210> 1
<211> 15
<212> PRT
<213> Artificial Sequence

<220>
<223> Dby peptide

<400> 1
Asn Ala Gly Phe Asn Ser Asn Arg Ala Asn Ser Ser Arg Ser Ser
1               5                   10                  15

<210> 2
<211> 9
<212> PRT
<213> Artificial Sequence

<220>
<223> Uty peptide

<400> 2
Trp Met His His Asn Met Asp Leu Ile
1               5

<210> 3
<211> 8
<212> PRT
<213> Artificial Sequence

<220>
<223> OVA257-264

<400> 3
Ser Ile Ile Asn Phe Glu Lys Leu
1               5

<210> 4
<211> 17
<212> PRT
<213> Artificial Sequence

<220>

<223> OVA323-339

<400> 4
Ile Ser Gln Ala Val His Ala Ala His Ala Glu Ile Asn Glu Ala Gly
1               5                   10                  15
Arg


<210> 5
<211> 20
<212> DNA
<213> Artificial Sequence


<220>
<223> 920hasarco.F

<400> 5
tgctggccta tgtcatgtgc                                                   20


<210> 6
<211> 20
<212> DNA
<213> Artificial Sequence


<220>
<223> 991hasarco.R

<400> 6
tctggatgtc ggaggtagcc                                                   20


<210> 7
<211> 25
<212> DNA
<213> Artificial Sequence


<220>
<223> 946hasarco.P

<400> 7
cgggagggaa ggctgaagag agacc                                             25


<210> 8
<211> 20
<212> DNA
<213> Artificial Sequence


<220>
<223> m18lPO.F

<400> 8
ctccaagcag atgcagcaga                                                   20


<210> 9
<211> 20
<212> DNA

<213> Artificial Sequence

<220>
<223> m267PO.R

<400> 9
accatgatgc gcaaggccat                                                    20

<210> 10
<211> 22
<212> DNA
<213> Artificial Sequence

<220>
<223> m225PO.P

<400> 10
ccgtggtgct gatgggcaag aa                                                 22

<210> 11
<211> 21
<212> DNA
<213> Artificial Sequence

<220>
<223> mTitin-F

<400> 11
aaaacgagca gtgacgtgag c                                                  21

<210> 12
<211> 20
<212> DNA
<213> Artificial Sequence

<220>
<223> mTitin-R

<400> 12
ttcagtcatg ctgctagcgc                                                    20

<210> 13
<211> 25
<212> DNA
<213> Artificial Sequence

<220>
<223> mTitin-P

<400> 13
tgcacggaag cgtctcgtct cagtc                                              25

<210> 14

<211> 20
<212> DNA
<213> Artificial Sequence

<220>
<223> mCMV-F

<400> 14
catcaatggg cgtggatagc                                                   20

<210> 15
<211> 24
<212> DNA
<213> Artificial Sequence

<220>
<223> mCMV-R

<400> 15
ggagttgtta cgacattttg gaaa                                              24

<210> 16
<211> 18
<212> DNA
<213> Artificial Sequence

<220>
<223> mCMV-P

<400> 16
atttccaagt ctccaccc                                                     18